# EUROPÄISCHE PATENTANMELDUNG

(11) **EP 3 135 260 A1**
(43) Veröffentlichungstag der Anmeldung: **01.03.2017**
(21) Anmeldenummer: 16184507.8
(22) Anmeldetag: 17.08.2016
(51) Int. Cl.: A61F 5/56

(54) **VORRICHTUNG ZUM EINSTELLEN EINER DEFINIERTEN STELLUNG EINES KIEFERS**

(30) Priorität: 17.08.2015 DE 102015215661
(71) Anmelder: Escherich, Rudolf, 81825 München (DE)
(72) Erfinder: Escherich, Rudolf, 81825 München (DE)
(74) Vertreter: Hahner, Ralph

(57) **Zusammenfassung**

Vorrichtung zum Einstellen einer definierten Stellung eines Kiefers, insbesondere zur Behandlung von schnarchen. Diese Vorrichtung weist eine Oberkiefereinrichtung (2), welche in der Weise ausgebildet ist, um wenigstens teilweise an Zähne eines Oberkiefers, insbesondere formschlüssig und/oder kraftschlüssig, gekoppelt zu werden und eine Unterkiefereinrichtung (3), welche in der Weise ausgebildet ist, um wenigstens teilweise mit Zähnen eines Unterkiefers, insbesondere formschlüssig und/oder kraftschlüssig, gekoppelt zu werden, auf. Dabei weisen die Oberkiefereinrichtung und die Unterkiefereinrichtung jeweils genau zwei Stütz-bereiche (11) auf, welche symmetrisch auf beiden Seiten eines Bereichs angeordnet sind, welcher etwa der Mitte des Kiefers entspricht. Insbesondere sind die Stütz-bereiche korrespondierend zum Abschnitt der Prämolaren des Kiefers angeordnet. Zudem sind jeweils ein Paar gegenüberliegender Stützbereiche (11a,b) der Oberkiefereinrichtung und der Unterkiefereinrichtung in der Weise ausgebildet, um wenigstens in der definierten Stellung des Kiefers aneinander anzugrenzen. Außerdem ist wenigstens eines der Paare, vorzugsweise zwei Paare, in der Weise ausgebildet, um sich wenigstens in der definierten Stellung des Kiefers magnetisch anzuziehen. Schließlich sind die Stützbereiche in der Weise ausgebildet, dass die Oberkiefereinrichtung und die Unterkiefereinrichtung bei aneinander angrenzenden Stützbereichen wenigstens im Wesentlichen entlang einer Hauptachse (HA) der Vorrichtung relativ zueinander verschiebbar sind

## Beschreibung

Die vorliegende Erfindung betrifft eine Vorrichtung zum Einstellen einer definierten Stellung eines Kiefers. Insbesondere dient die Vorrichtung zur Behandlung von Schnarchen.

Die Stellung eines Kiefers, also die relative Position von Unterkiefer und Oberkiefer zueinander, beeinflusst die Kaumuskulatur, die Nutzung der Zähne des Unterkiefers und des Oberkiefers, die umliegende Gesichtsmuskulatur sowie Nervenaktivitäten, die Öffnung des Mundes, die Lage der Zunge und die Form des Rachens. Durch Fehlstellungen des Kiefers kann es zu Kopfschmerzen, Ohrgeräuschen, Abnutzung der Zähne, Verspannung der Nackenmuskulatur, Atemproblemen oder Schnarchen kommen.

Derartige Symptome lassen sich durch Einstellen einer definierten Stellung des Kiefers behandeln. Dazu kann etwa eine gewünschte, definierte Stellung eines Kiefers durch eine Vorrichtung direkt vorgegeben und/oder neuromuskulär trainiert werden. Auf diese Weise kann insbesondere der Unterkiefer gegenüber dem Oberkiefer in einer gewünschten Stellung gehalten werden und anforderungsabhängig zusätzlich eine Kraft auf Teile des Kiefers ausgeübt werden.

Insbesondere das Schnarchen kann sowohl ein gesellschaftliches als auch ein gesundheitliches Problem darstellen. Allgemein kann Schnarchen bei einem schlafenden Menschen auftreten und von anderen Menschen, etwa wenn diese ebenfalls schlafen möchten, als störend empfunden werden. Darüber hinaus kann je nach Ausprägung des Schnarchens lediglich die Schlafqualität leicht beeinträchtigt sein, der Hals durch das Schnarchen belastet sein oder die Schlafqualität stark leiden und weitere gesundheitliche Probleme wie Tagesmüdigkeit, Bluthochdruck und ein erhöhtes Risiko für Schlaganfall oder Herzinfarkt auftreten.

Schnarchen kann durch eine flatternde Bewegung des Gaumensegels im Sog der Atemluft entstehen. Insbesondere im Schlaf entspannt sich die Rachenmuskulatur, wodurch sich der Atemweg am Gaumensegel entlang verkleinern und das Gaumensegel leichter zu flatternde Bewegungen angeregt werden kann. Zudem kann bei einer Atmung durch den Mund, etwa wenn durch die Nase aufgrund angeschwollener Nasenschleimhäute keine Atmung möglich ist, ein Zurückfallen der Zunge in den Rachen den Atemweg dort verengen und das Risiko oder die Ausprägung des Schnarchens weiter vergrößern.

Zur Behandlung von Schnarchen mittels Einstellen einer definierten Stellung eines Kiefers kann der Unterkiefer gegenüber dem Oberkiefer so positioniert werden, dass entweder der Mund geschlossen wird und dadurch eine Atmung durch die Nase erzwungen oder der Mund offengehalten wird und dadurch die Atmung durch den Mund unterstützt wird. Speziell eine Protrusion des Unterkiefers, d.h. bezogen auf einen Menschen ein Nach-Vorne-Schieben seines Unterkiefers, bewegt auch die Zunge in protrusiver Richtung und schafft so mehr Raum im Rachen.

Aus der DE 3 625 790 C2 ist eine Vorrichtung zum Verhindern des menschlichen Schnarchens, eine so genannte Schnarchschiene, bekannt. Ziel der Vorrichtung ist es, das Öffnen des Mundes des Benutzers im Schlaf zu unterbinden, wobei die schlafende Person dadurch gehalten wird, durch die Nase zu atmen.

Die US 2014/0072927 A1 beschreibt eine Vorrichtung zur Protrusion eines Unterkiefers, um den Mund zu öffnen und die Atmung durch den Mund zu unterstützen.

Auch die CN 1023191319 A offenbart eine obere Platte und eine untere Platte, um den Mund geöffnet zu halten und den Unterkiefer gegenüber dem Oberkiefer in protrusiver Richtung zu verschieben.

Es ist eine die Aufgabe der Erfindung, eine verbesserte Vorrichtung zum Einstellen einer definierten Stellung eines Kiefers zur Verfügung zu stellen. Insbesondere liegt der Erfindung die Aufgabe zugrunde, eine Vorrichtung zur Behandlung von Schnarchen, insbesondere bei einem schlafenden Menschen, zur Verfügung zu stellen. Speziell ist es wünschenswert, den Tragekomfort, den erzielten Behandlungseffekt, die Haltbarkeit oder die Hygiene zu verbessern.

Diese Aufgabe wird durch eine Vorrichtung gemäß der Lehre des unabhängigen Anspruchs 1 gelöst. Vorteilhafte Ausgestaltungen der Erfindung sind Gegenstand der Unteransprüche. Die Ansprüche werden mit dieser ausdrücklichen Bezugnahmen zum Inhalt der Beschreibung gemacht.

Ein erster Aspekt der Erfindung betrifft eine Vorrichtung zum Einstellen einer definierten Stellung eines Kiefers, insbesondere zur Behandlung von Schnarchen. Die erfindungsgemäße Vorrichtung weist eine Oberkiefereinrichtung, welche in der Weise ausgebildet ist, um wenigstens teilweise an Zähne eines Oberkiefers, insbesondere formschlüssig und/oder kraftschlüssig, gekoppelt zu werden und eine Unterkiefereinrichtung, welche in der Weise ausgebildet ist, um wenigstens teilweise mit Zähnen eines Unterkiefers, insbesondere formschlüssig und/oder kraftschlüssig, gekoppelt zu werden, auf. Dabei weisen die Oberkiefereinrichtung und die Unterkiefereinrichtung jeweils genau zwei Stützbereiche auf, welche, insbesondere im Wesentlichen symmetrisch, auf beiden Seiten eines Bereichs angeordnet sind, welcher etwa der Mitte des Kiefers entspricht. Insbesondere sind die Stützbereiche korrespondierend zum Abschnitt der Prämolaren des Kiefers angeordnet. Zudem sind jeweils ein Paar gegenüberliegender Stützbereiche der Oberkiefereinrichtung und der Unterkiefereinrichtung in der Weise ausgebildet, um wenigstens in der definierten Stellung des Kiefers aneinander anzugrenzen. Außerdem ist wenigstens eines der Paare, vorzugsweise beide Paare, in der Weise ausgebildet, um sich wenigstens in der definierten Stellung des Kiefers magnetisch anzuziehen. Schließlich sind die Stützbereiche in der Weise ausgebildet, dass die Oberkiefereinrichtung und die Unterkiefereinrichtung bei aneinander angrenzenden Stützbereichen wenigstens im Wesentlichen entlang einer Hauptachse der Vorrichtung relativ zueinander verschiebbar sind.

Für die Beschreibung von Richtungen, Bereichen und Positionen in Bezug auf den Kiefer wird insbesondere auf die dem Fachmann bekannten Bezeichnungen zurückgegriffen. Dabei können sich diese Bezeichnungen der Einfachheit halber auch direkt auf den Kiefer beziehen. Die jeweils beschriebene Richtung, der jeweils beschriebene Bereich oder die jeweils beschriebene Position der Vorrichtung ist vorzugsweise korrespondierend zu der Richtung, dem Bereich bzw. dem Abschnitt oder der Position beim Kiefer zu verstehen, wenn die Vorrichtung eingesetzt ist, also die Oberkiefereinrichtung an die Zähne des Oberkiefers und/oder die Unterkiefereinrichtung an die Zähne des Unterkiefers gekoppelt ist.

Sofern bei der Beschreibung einer Richtung auf einen oder mehrere Zähne Bezug genommen wird, so ist eine Richtung zu verstehen, die zumindest im Wesentlichen der jeweiligen Richtung bei dem Zahn oder den Zähnen entspricht. Insbesondere soll der Winkel, welcher von der beschriebenen Richtung und der jeweiligen Richtung einer Normalstellung des Zahns im Kiefer eingeschlossen wird, vorzugsweise kleiner als 5° sein. Die Ausrichtung verschiedener Zähne weicht im Allgemeinen voneinander ab und bei Bezugnahme auf wenigstens zwei Zähne ist unter einer auf diese Weise beschriebenen Richtung vorzugsweise eine Richtung zu verstehen, welche der jeweiligen Richtung von wenigstens einem der Zähne entspricht. Bevorzugt handelt es sich bei dieser Richtung um eine mittlere oder kombinierte Richtung in Bezug auf die wenigstens zwei Zähne.

Unter einer definierten Stellung eines Kiefers ist im Sinne der Erfindung zu verstehen, dass ein Unterkiefer des Kiefers und ein Oberkiefer des Kiefers relativ zueinander eine definierte, bestimmte oder insbesondere erwünschte Position haben. Insbesondere ein Festhalten des Unterkiefers mit einer Kraft relativ zum Oberkiefer in protrusiver Richtung, in dorsaler Richtung, in okklusaler Richtung oder gegen die okklusale Richtung, also ein Öffnen des Kiefers, in dextrale Richtung, in sinistrale Richtung, ein geöffneter Kiefer, ein geschlossener Kiefer oder ein Kiefer in Kopfbiss-Stellung ist eine definierte Stellung eines Kiefers.

Eine Oberkiefereinrichtung im Sinne der Erfindung ist eine Einrichtung, welche in der Weise ausgebildet ist, um wenigstens teilweise an Zähne eines Oberkiefers, insbesondere formschlüssig und/oder kraftschlüssig, gekoppelt zu werden. Insbesondere kann eine Oberkiefereinrichtung aus Kunststoff, Tiefziehfolie und/oder Metall gefertigt sein. Zur Kopplung an die Zähne des Oberkiefers kann die Form der Oberkiefereinrichtung zumindest teilweise der Form der Zähne entsprechen, um eine formschlüssige Kopplung zu ermöglichen. Speziell kann die Oberkiefereinrichtung eine feste Kunststoffschiene aufweisen und ein thermoplastisches Material, welches durch Erwärmen, etwa auf 155 °C, bei einer Aufheizdauer von etwa 1,5 Minuten plastisch wird und mittels Anpressen an die Zähne des Oberkiefers und Abkühlenlassen in der Weise formbar ist, um eine formschlüssige Kopplung an die Zähne des Oberkiefers zu ermöglichen. Auch kann die Oberkiefereinrichtung zumindest teilweise so ausgeformt sein, dass diese beim Tragen bzw. beim Koppeln an die Zähne des Oberkiefers reversibel deformiert wird und eine Kraft auf die Zähne des Oberkiefers bewirkt, woraus eine kraftschlüssige Verbindung folgt. Allgemein erfolgt das Einsetzen der Oberkiefereinrichtung mittels einer Bewegung entgegen der okklusalen Richtung, so dass eine formschlüssige und/oder kraftschlüssige Kopplung besteht und keine weitere Bewegung entgegen der okklusalen Richtung möglich ist; umgekehrt erfolgt im Allgemeinen das Entnehmen der eingesetzten Oberkiefereinrichtung mittels einer Bewegung in okklusaler Richtung. Auch wird im Allgemeinen durch die formschlüssige und/oder kraftschlüssige Kopplung eine Bewegung der eingesetzten Oberkiefereinrichtung in dorsaler Richtung, in protrusiver Richtung, in bukkaler bzw. labialer Richtung oder entgegen der bukkalen bzw. labialen Richtung verhindert. Insbesondere sind folglich räumliche Bereiche der Oberkiefereinrichtung korrespondierenden Abschnitten des Oberkiefers fest zugeordnet. Überdies kann die Oberkiefereinrichtung aus einem Teil oder aus mehreren Teilen bestehen, welche miteinander flexibel, trennbar, starr oder nicht verbunden sind. Schließlich ist eine Oberkieferschiene, eine Oberkieferspange oder eine Zahnspange für den Oberkiefer eine Oberkiefereinrichtung im Sinne der Erfindung.

Eine Unterkiefereinrichtung im Sinne der Erfindung ist eine Einrichtung, welche vergleichbar mit einer Oberkiefereinrichtung aber für den Unterkiefer ausgebildet ist.

Ein Stützbereich im Sinne der Erfindung ist ein räumlich abgegrenzter Bereich einer Vorrichtung oder eines Teils einer Vorrichtung, welcher in der Weise ausgebildet ist, um die Vorrichtung bzw. den Teil der Vorrichtung gegenüber einer weiteren Vorrichtung bzw. einen weiteren Teil der Vorrichtung abzustützen. Dazu ist die Vorrichtung bzw. der Teil der Vorrichtung relativ zu der weiteren Vorrichtung bzw. dem weiteren Teil der Vorrichtung in wenigstens eine Position bringbar, so dass der Stützbereich an die weitere Vorrichtung bzw. den weiteren Teil der Vorrichtung angrenzt, insbesondere an wenigstens einen weiteren Stützbereich. Insbesondere können der Stützbereich und der wenigstens eine weitere Stützbereich als ein Paar zusammengefasst werden. Zudem kann das Paar so ausgebildet sein, dass sich der Stützbereich und der weitere Stützbereich gegenüberliegen, insbesondere auch wenn diese nicht aneinander angrenzen. Schließlich kann ein Stützbereich wenigstens eine Stützfläche aufweisen, wobei insbesondere bei einem Paar aneinander angrenzender Stützbereiche auch die entsprechenden wenigstens einen Stützflächen aneinander angrenzen.

Eine Hauptachse der Vorrichtung im Sinne der Erfindung ist eine Achse, die durch einen Bereich, welcher etwa der Mitte des Kiefers entspricht, verläuft und mit der okklusalen Richtung einen Winkel von insbesondere 90° einschließt. Dabei entspricht die okklusale Richtung insbesondere der okklusalen Richtung bezogen auf einen Backenzahn und/oder Prämolar des Kiefers. Insbesondere verläuft die Hauptachse der Vorrichtung in protrusiver Richtung bzw. entgegen der dorsalen Richtung. Dies entspricht zumindest im Wesentlichen insbesondere der mesialen bzw. distalen Richtung bezogen auf einen Backenzahn, insbesondere bezogen auf einen großen Backenzahn.

Ein Vorteil der erfindungsgemäßen Vorrichtung liegt in der Verschiebbarkeit der Oberkiefereinrichtung und der Unterkiefereinrichtung wenigstens im Wesentlichen entlang der Hauptachse bei aneinander angrenzenden Stützbereichen. Auf diese Weise wird zwar die definierte Stellung des Kiefers vorgegeben aber eine Bewegung aus dieser heraus ermöglicht. Dabei wird die mögliche Bewegung aus der definierten Stellung heraus insbesondere durch die Hauptachse vorgegeben. Bevorzugt lassen sich so unerwünschte Bewegungen verhindern. Zudem lässt sich durch die Verschiebbarkeit eine Zwangsstellung vermeiden, was den Tragekomfort erhöht. Außerdem kann dadurch die Haltbarkeit der Vorrichtung erhöht werden, da der Kiefer bzw. seine Muskulatur beim Tragen der Vorrichtung nicht reflexartig gegen die Zwangsstellung arbeitet.

Ein weiterer Vorteil ergibt sich aus der magnetischen Anziehung wenigstens eines Paares der Stützbereiche. Damit lässt sich eine einfache und haltbare Konstruktion, insbesondere ohne aufwändige Mechanik, erreichen. Auch kann auf diese Weise die Hygiene der Vorrichtung und ihr Tragekomfort verbessert werden. Außerdem erlaubt die magnetische Anziehung zahlreiche Anpassungsmöglichkeiten sowie Erweiterungen der Vorrichtung.

Mittels der erfindungsgemäßen Vorrichtung lassen sich über die an den Oberkiefer gekoppelte Oberkiefereinrichtung und über die an den Unterkiefer gekoppelte Unterkiefereinrichtung der Oberkiefer unter Unterkiefer relativ zueinander auf vorteilhafte Weise direkt positionieren, wodurch die definierte Stellung des Kiefers vorgegeben wird. Dies kann insbesondere zum erzielten Behandlungserfolg beitragen und/oder den Tragekomfort.

Überdies stützen die Stützbereiche wenigstens in der definierten Stellung des Kiefers die Oberkiefereinrichtung und die Unterkiefereinrichtung und damit in Oberkiefer und den Unterkiefer gegeneinander auf vorteilhafte Weise ab. Die Anordnung der Stützbereiche kann den Kiefer und hier speziell das Kiefergelenk sowie die Kiefermuskulatur entlasten. Bevorzugt sind die jeweils genau zwei Stützbereiche in der Weise angeordnet, um zumindest im Wesentlichen bei den genau zwei Bereichen zu liegen, welche mit dem jeweiligen Hauptkauzentrum, also dem dextralen Hauptkauzentrum und dem sinistralen Hauptkauzentrum, korrespondieren. Bei einem erwachsenen Menschen liegt das jeweilige Hauptkauzentrum etwa im Abschnitt der Prämolaren; insbesondere gemäß dem FDI-Zahnschema liegt das dextrale Hauptkauzentrum bei den Zähnen 14 bis 16 des Oberkiefers und 44 bis 46 des Unterkiefers sowie das sinistrale Hauptkauzentrum bei den Zähnen 24 bis 26 des Oberkiefers und 34 bis 36 des Unterkiefers. Bei einem Kind das jeweilige Hauptkauzentrum entsprechend der geringeren Anzahl an Zähnen verschoben. Insbesondere liegen das dextralen Hauptkauzentrum und das sinistralen Hauptkauzentrum bei eingesetzter Vorrichtung im Wesentlichen symmetrisch zu der Hauptachse der Vorrichtung.

Durch das Abstützen lediglich im jeweiligen Hauptkauzentrum und die symmetrische Anordnung wird der Kiefer und hier speziell das Kiefergelenk sowie die Kiefermuskulatur entlastet. Dies ist insbesondere gegenüber solchen bestehenden Systemen vorteilhaft, welche mehrere Auflagepunkte bzw. Stützbereiche pro Kieferseite aufweisen, da bei diesen die Stellung des Kiefergelenks in einer einzigen Winkelposition, insbesondere einer Zwangsstellung, festgelegt ist. Dies führt zu einer Belastung des Kiefergelenks. Bevorzugt ist der Oberkiefer gegenüber dem Unterkiefer über die Unterkiefereinrichtung daher nicht nur abgestützt sondern auch um eine Achse gelagert, welche durch die beiden Hauptkauzentren verläuft und insbesondere zumindest im Wesentlichen senkrecht zur Hauptachse der Vorrichtung ist. Diese Lagerung kann die Entlastung des Kiefers noch weiter unterstützen.

Insgesamt lassen sich insbesondere mit der vorteilhaften Kombination von Verschiebbarkeit, magnetischer Anziehung und Kopplung der Oberkiefereinrichtung und Unterkiefereinrichtung sowie der Anordnung der Stützbereiche ein erhöhter Tragekomfort, ein verbesserter erzielbare Behandlungseffekt und eine verlängerte Haltbarkeit sowie eine verbesserte Hygiene erreichen. Insbesondere wird eine Belastung des Kiefers aufgrund einer Zwangsstellung und damit Verspannungen und Schmerzen der Kiefermuskulatur sowie des Kiefergelenks vermieden. Speziell wird der Unterkiefer gegenüber dem Oberkiefer mittels der erfindungsgemäßen Vorrichtung so abgestützt und gelagert sowie zugleich in die definierte Stellung gebracht ohne diese Stellung starr, d.h. ohne Bewegungsmöglichkeit, vorzugeben, dass sich die Kiefermuskulatur entspannen und das Kiefergelenk nicht belastet wird.

Nachfolgend werden vorteilhafte Ausgestaltungen der Vorrichtung beschrieben, jeweils, soweit dies technisch möglich ist und nicht ausdrücklich ausgeschlossen wird, beliebig miteinander kombiniert werden können.

Gemäß einer vorteilhaften Ausgestaltung der Vorrichtung sind die Stützbereiche in der Weise, insbesondere okklusal, auf der Oberkiefereinrichtung und der Unterkiefereinrichtung ausgebildet, um in der definierten Stellung des Kiefers in einem Zwischenraum zwischen Kauflächen des Oberkiefers und Kauflächen des Unterkiefers aneinander anzugrenzen. Da die Stützbereiche in dem Zwischenraum zwischen den Kauflächen angeordnet sind, werden weder die Innenseite des Mundes noch die Zunge durch die Stützbereiche beeinträchtigt. Insbesondere könnten die Stützbereiche bzw. Stützflächen oder Stützeinrichtungen der Stützbereiche einen Nutzer stören oder bei regelmäßigem Kontakt mit Gewebe sogar Entzündungen hervorrufen.

Gemäß einer weiteren vorteilhaften Ausgestaltung der Vorrichtung sind die Stützbereiche in jeweils einem Bereich angeordnet, welcher einem Abschnitt der Prämolaren des Kiefers entspricht. Die Prämolaren sind dabei jene Zähne, welche insbesondere auf beiden Seiten des Bereichs, welcher etwa der Mitte des Kiefers entspricht, angeordnet sind.

Gemäß einer weiteren vorteilhaften Ausgestaltung der Vorrichtung ist eine magnetische Anziehungskraft des wenigstens einen Paars gegenüberliegender Stützbereiche in der Weise eingerichtet, um eine Verschiebung und/oder eine Trennung der Oberkiefereinrichtung und der Unterkiefereinrichtung durch Muskelkraft, insbesondere der Kiefermuskulatur, zu ermöglichen. Insbesondere wird darauf abgezielt, die magnetische Anziehungskraft physiologisch an die Muskulatur, insbesondere die Kiefermuskulatur, anzupassen Auf diese vorteilhafte Weise lässt sich erreichen, dass der Kiefer auch geöffnet werden kann, wenn die Vorrichtung getragen wird. Dies erhöht insbesondere den Tragekomfort. Auch, wie vorausgehend bereits beschrieben, lässt sich mittels der Verschiebbarkeit ein erhöhter Tragekomfort erzielen und eine Zwangsstellung vermeiden.

Gemäß einer weiteren vorteilhaften Ausgestaltung weist die magnetische Anziehung zwischen der Oberkiefereinrichtung und der Unterkiefereinrichtung insgesamt eine Abzugskraft zwischen 0,1 N und 50 N, bevorzugt zwischen 1 N und 30 N, weiter bevorzugt zwischen 5 N und 15 N insbesondere von etwa 12 N auf, wenn sich aneinander angrenzende Stützbereiche des wenigstens einen Paares sich anziehender Stützbereiche voll abdecken. Die hier gewählten Werte für die magnetische Anziehung erlauben es, wie physiologisch erforscht worden ist, die Oberkiefer und die Unterkiefereinheit voneinander zu trennen und/oder relativ zueinander zu verschieben. Insbesondere werden die dazu erforderlichen Kräfte von einem Menschen als angenehm empfunden. So ist etwa einerseits kein übermäßiger Kraftaufwand dazu nötig und andererseits muss dennoch eine gewisse Kraft überwunden werden, so dass ein versehentliches Trennen und/oder Verschieben vermieden werden kann. Überdies lässt sich mit der hier gewählten magnetischen Anziehung erreichen, dass beim Trennen und/oder Verschieben die Oberkiefereinrichtung bzw. die Unterkiefereinrichtung an den Zähnen des Oberkiefers bzw. Unterkiefers gekoppelt bleiben.

Gemäß einer weiteren vorteilhaften Ausgestaltung der Vorrichtung sind beide Paare aneinander angrenzender Stützbereiche in der Weise ausgebildet, um die Oberkiefereinrichtung und die Unterkiefereinrichtung gegeneinander abzustützen. Dabei wird die Oberkiefereinrichtung in einer okklusalen Richtung, insbesondere bezogen auf einen Backenzahn des Oberkiefers, und die Unterkiefereinrichtung in einer okklusalen Richtung, insbesondere bezogen auf einen Backenzahn Unterkiefers, abgestützt. Ein Vorteil der einen weiteren vorteilhaften Ausgestaltung ist insbesondere, dass für den Kiefer eine Mindestöffnung für seine definierte Stellung vorgegeben wird. Insbesondere kann der Kiefer sich in diesem Fall nicht vollständig schließen, was eine Atmung durch den Mund unterstützt. Um eine definierte Stellung des Kiefers einzustellen, muss insbesondere diese Stellung fest vorgegeben werden und/oder Kräfte in der Weise wirken, dass diese in Richtung der definierten Stellung des Kiefers wirken und somit das Einstellen der definierten Stellung des Kiefers wenigstens so weit unterstützen, dass dies spürbar ist und geringere Widerstände, etwa durch Reibung oder unbewusste Muskelanspannungen, überwindet. Hierbei ist es insbesondere vorteilhaft, die Bewegung in der okklusalen Richtung mechanisch einzuschränken, da die Kiefermuskulatur in dieser Richtung die größten Kräfte entfaltet.

Gemäß einer weiteren vorteilhaften Ausgestaltung der Vorrichtung sind beide Paare aneinander angrenzender Stützbereiche in der Weise ausgebildet sind, um die Oberkiefereinrichtung und die Unterkiefereinrichtung in einem definierten Abstand, welcher etwa 10 mm bis 2 mm, bevorzugter 8 mm bis 4 mm, und am bevorzugtesten 6 mm beträgt, gegeneinander abzustützen. Ein ausreichender Abstand zwischen unterem Rand der Oberkiefereinrichtung und oberem Rand der Unterkiefereinrichtung gewährleistet, dass eine Öffnung, durch welche Atemluft durch die erfindungsgemäße Vorrichtung in den Rachen eines Nutzers einströmen kann, genügend groß ist, um eine Luftversorgung des Nutzers durch den Mund zu gewährleisten. Des Weiteren wird hierdurch eine erfindungsgemäß gewünschte, offene Stellung des Rachens begünstigt.

Gemäß einer weiteren vorteilhaften Ausgestaltung weist die Vorrichtung bei aneinander angrenzenden Stützbereichen wenigstens eine Öffnung für Atemluft auf. Dabei ist in einer bevorzugten Variante die wenigstens eine Öffnung für Atemluft in einem Bereich ausgebildet, welcher wenigstens im Wesentlichen einem Abschnitt zwischen den Schneidezähnen des Oberkiefers und des Unterkiefers entspricht. Überdies ist in einer bevorzugten Variante die wenigstens eine Öffnung zwischen der Oberkiefereinrichtung und der Unterkiefereinrichtung ausgebildet. Auf diese vorteilhafte Weise lässt sich eine Atmung durch den Mund erreichen. Bevorzugt wird die wenigstens eine Öffnung für Atemluft dabei möglichst groß ausgeführt, um den Widerstand beim Atmen durch die wenigstens eine Öffnung zu reduzieren und so die Atmung durch den Mund zu erleichtern. Der möglichen maximalen Größe wenigstens einen Öffnung für Atemluft wirken insbesondere die Abmessungen der Oberkiefereinrichtung und der Unterkiefereinrichtung entgegen. Folglich wird die Oberkiefereinrichtung und/oder die Unterkiefereinrichtung in einer bevorzugten Variante dem Bereich, welcher für die wenigstens eine Öffnung für Atemluft vorgesehen ist, möglichst zierlich ausgebildet. Insbesondere kann hierzu mit einer reduzierten Materialstärke und/oder Aussparungen gearbeitet werden. Schließlich ist es bevorzugt die Oberkiefereinrichtung und die Unterkiefereinrichtung in diesem Bereich nur soweit auszuformen, wie dies für die Koppelung an den Kiefer erforderlich ist. Schließlich lässt sich, speziell wenn eine Atmung durch die Nase erschwert ist, mittels der wenigstens einen Öffnung für Atemluft ein verbesserter Effekt bei der Behandlung von Schnarchen erzielen.

Gemäß einer weiteren vorteilhaften Ausgestaltung der Vorrichtung weisen die Stützbereiche jeweils wenigstens eine Stützfläche auf, die jeweils auf Stützeinrichtungen angeordnet sind. Auf diese vorteilhafte Weise lassen sich die definierte Stellung des Kiefers, mögliche Richtungen für eine Verschiebung aus der definierten Stellung des Kiefers heraus und Richtungen, in die aufgrund aneinander angrenzender Stützflächen keine Verschiebung möglich ist, gezielt definieren.

Gemäß einer weiteren vorteilhaften Ausgestaltung der Vorrichtung weisen die Stützbereiche jeweils wenigstens eine Stützfläche auf. Zudem sind die Oberkiefereinrichtung und die Unterkiefereinrichtung bei aneinander angrenzenden Stützbereichen relativ zueinander verschiebbar. Dabei sind diese zumindest im Wesentlichen in einer Ebene, welche durch die Stützflächen definiert wird, ausgehend von der definierten Stellung des Kiefers um etwa 1 mm, bevorzugt um wenigstens 5 mm und weiter bevorzugt um wenigstens 15 mm verschiebbar sind. In einer bevorzugten Variante liegt die Ebene und/oder verläuft die Verschiebung mit wenigstens einer Richtungskomponente entlang der Hauptachse und/oder mit wenigstens einer Richtungskomponente senkrecht zur Hauptachse. Es ergeben sich insbesondere wieder die Vorteile der Verschiebbarkeit. Speziell werden dabei die Vorteile durch die wenigstens erzielbaren Strecken für die Verschiebung weiter verbessert. Schließlich lässt sich insbesondere durch die erlaubten Verschiebestrecken ein erhöhter Tragekomfort erzielen.

Gemäß einer weiteren vorteilhaften Ausgestaltung sind die Oberkiefereinrichtung und die Unterkiefereinrichtung wenigstens in beide Richtungen entlang der Hauptachse relativ zueinander verschiebbar. Aus der Verschiebbarkeit in beide Richtungen ergibt sich ein verbesserter Tragekomfort. Insbesondere bei einer definierten Stellung des Kiefers, welche eine Verschiebung des Kiefers aus seiner Ruheposition zumindest teilweise in Richtung der Hauptachse verlangt, ist die Verschiebbarkeit beide Richtungen entlang der Hauptachse wichtig, um eine Zwangsstellung und hierbei speziell Kiefergelenksschmerzen zu vermeiden. Vorzugsweise sind die Oberkiefereinrichtung und die Unterkiefereinrichtung ausgehend von der definierten Stellung des Kiefers um 1 mm, bevorzugt um wenigstens 5 mm und weiter bevorzugt um wenigstens 15 mm relativ zueinander verschiebbar.

Gemäß einer weiteren vorteilhaften Ausgestaltung sind an dem wenigstens einen Paar sich anziehender Stützbereiche jeweils Magnetelemente in der Weise angeordnet, um zwischen der Oberkiefereinrichtung und der Unterkiefereinrichtung eine Kraft in Richtung der definierten Stellung des Kiefers zu bewirken. Ein Vorteil dieser Ausgestaltung ist es insbesondere, dass der Kiefer über die magnetische Kraft in die definierte Stellung gebracht werden kann. Dabei ist es insbesondere möglich, die definierte Stellung des Kiefers, etwa durch Muskelkraft, wieder zu verlassen. Zudem lässt sich ein automatisches Einstellen der definierten Stellung des Kiefers erzielen, sobald keine Kraft mehr gegen dieses Einstellen aufgewandt wird. Auch wird das Einstellen in die für die Behandlung gewünschte, definierte Stellung des Kiefers intuitiv unterstützt. Hierdurch wird insbesondere der Tragekomfort verbessert und/oder der Behandlungseffekt erhöht. Des Weiteren lassen sich übermäßige mechanische Kräfte auf diese vorteilhafte Weise vermeiden und die Haltbarkeit der Vorrichtung verlängern. Schließlich sind kann auf mechanische Komponenten für das Einstellen verzichtet werden, was die Hygiene verbessern kann. Vorzugsweise sind die Magnetelemente jeweils vollständig von der der Oberkiefereinrichtung oder der Unterkiefereinrichtung umschlossen. Hierdurch kann vermieden werden, dass die Magnetelemente aufeinander reiben oder durch Körperflüssigkeiten angegriffen werden. Insbesondere kann gewährleistet werden, dass eine Eloxierbeschichtung auf den Magnetelementen nicht beschädigt wird.

Ein Magnetelement im Sinne der Erfindung ist eine Einheit, welche magnetische Eigenschaften hat. Dabei kann das Magnetelement insbesondere permanent-magnetische, ferrimagnetische oder ferromagnetische Substanzen oder Untereinheiten aufweisen. Weiterhin können sich zwei Magnetelemente magnetisch anziehen oder abstoßen oder allgemein eine magnetische Kraft zwischen den beiden Magnetelemente wirken. Insbesondere sind Permanentmagnete wie Neodym-Magnete oder Einheiten aus einem ferromagnetischen Material wie Eisen oder Stahl, insbesondere Eisenblöcke, Magnetelemente im Sinne der Erfindung.

Gemäß einer weiteren vorteilhaften Ausgestaltung weist jedes Paar sich anziehender Stützbereiche wenigstens ein Magnetelement auf, welches einen Permanentmagneten aufweist. Auf diese vorteilhafte Weise lässt sich eine magnetische Anziehung ohne Energieaufwand erzielen, darüber hinaus kann der jeweils andere Stützbereich lediglich ein Magnetelement mit ferromagnetischen Eigenschaften aufweisen, wobei mittels der Magnetisierung durch den Permanentmagneten eine magnetische Anziehung bewirkt wird. In einer bevorzugten Variante weisen beide Bereiche eines sich anziehenden Paars wenigstens einen Permanentmagneten auf. So lässt sich eine erhöhte magnetische Anziehung erzielen. In einer weiteren bevorzugten Variante weist nur ein Stützbereich eines sich anziehenden Paars einen Permanentmagneten auf und der andere Stützbereich ein ferromagnetisches Material, welches eine größere räumliche Ausdehnung bezogen auf die Stützbereiche hat als der Permanentmagnet. Insbesondere liegt ein Vorteil darin, dass der Permanentmagnet über diese größere räumliche Ausdehnung verschiebbar ist und eine Kraft, die der Verschiebung entgegenwirkt, erst auftritt, wenn die Verschiebung so groß ist, dass der Permanentmagnet nicht mehr vollständig von dem ferromagnetischen Material abgedeckt wird. Hierdurch lässt sich insbesondere ein räumlicher Bereich und nicht nur eine einzelne Position für die definierte Stellung des Kiefers vorgegeben.

Gemäß einer weiteren vorteilhaften Ausgestaltung ist die magnetische Anziehung zwischen allen Paaren sich anziehender Stützbereiche zumindest im Wesentlichen gleich groß. Ein Vorteil dieser Ausgestaltung liegt in der gleichmäßigen Kraftverteilung. Dies kann den Tragekomfort erhöhen und dem Behandlungseffekt verbessern.

Gemäß einer weiteren vorteilhaften Ausgestaltung ist der von wenigstens einem Magnetelement aufgewiesene Permanentmagnet in okklusaler Richtung, insbesondere bezogen auf einen Backenzahn oder Prämolar des Kiefers, polarisiert. Durch die Polarisierung in okklusaler Richtung lässt sich bei jeder Kieferstellung eine magnetische Anziehung realisieren. Insbesondere bei zwei in okklusaler Richtung polarisierten, Permanentmagneten eines Paares sich gegenüberliegender Stützflächen kann unabhängig von einer Verschiebung entlang der Hauptachse immer eine magnetische Anziehung sichergestellt werden, wenn sich zumindest in der definierten Stellung des Kiefers der Südpol des einen Permanentmagneten und der Nordpol des anderen Permanentmagneten gegenüberliegen.

Gemäß einer weiteren vorteilhaften Ausgestaltung weisen die Stützbereiche jeweils wenigstens eine Stützfläche und jedes Paar sich anziehender Stützbereiche wenigstens einen Permanentmagnet auf. Dabei ist die Position des wenigstens einen Permanentmagneten in einer durch die jeweilige wenigstens eine Stützfläche definierten Ebene verstellbar. Insbesondere ist dieser verstellbar in einer Richtung, welche wenigstens eine Komponente in Richtung der Hauptachse und/oder wenigstens in Richtung senkrecht zu dieser und/oder wenigstens in mesialer Richtung und/oder wenigstens in distaler Richtung aufweist. Auf diese vorteilhafte Weise lässt sich erreichen, dass die durch die erfindungsgemäße Vorrichtung vorgegebene definierte Stellung des Kiefers mittels Verstellen des wenigstens einen Permanentmagneten anpassbar ist. Insbesondere ist diese vorteilhafte Ausgestaltung auch nach ihrer Fertigung noch anpassbar und speziell feinjustierbar. Dies kann insbesondere den Tragekomfort und/oder Behandlungseffekt steigern.

Gemäß einer weiteren vorteilhaften Ausgestaltung weisen die Oberkiefereinrichtung und/oder die Unterkiefereinrichtung wenigstens eine Einstelleinrichtung, insbesondere eine Stellschraube, zum Verstellen der Position des wenigstens einen Magnetelements auf. In einer bevorzugten Variante ist die Stellschraube aus Titan gefertigt. Über die Einstelleinrichtung lässt sich die Position des wenigstens einen Magnetelements vorgeben. Insbesondere mittels einer Stellschraube kann so die gewünschte Position exakt eingestellt werden. Darüber hinaus lässt sich die Position auch erneut anpassen. So ist insbesondere eine iterative Anpassung bzw. Justierung der Position und damit der eingestellten definierten Stellung des Kiefers möglich.

Gemäß einer weiteren vorteilhaften Ausgestaltung weist die Oberkiefereinrichtung und/oder die Unterkiefereinrichtung wenigstens eine formschlüssige Führung, zur Führung des wenigstens einen Magnetelements auf. In einer bevorzugten Variante ist die formschlüssige Führung als Schiene oder Kasten bzw. Gehäuse ausgebildet. Mittels der formschlüssigen Führung lassen sich die möglichen Positionen des wenigstens einen verstellbaren Magnetelements vorgeben. Insbesondere kann mit der formschlüssigen Führung eine Bewegung in eine unerwünschte Richtung, etwa in okklusaler Richtung oder entgegen der okklusalen Richtung verhindert werden. Dies erhöht insbesondere die Haltbarkeit der Vorrichtung gemäß dieser weiteren vorteilhaften Ausgestaltung.

Gemäß einer weiteren vorteilhaften Ausgestaltung umschließt die Führung das wenigstens eine Magnetelement bis auf eine Öffnung für die Einstelleinrichtung vollständig. Auch hierdurch wird eine Beschädigung oder ein Angriff des Magnetmaterials durch Körperflüssigkeiten vermieden.

Gemäß einer weiteren vorteilhaften Ausgestaltung ist die Führung und die Oberkiefereinrichtung oder die Unterkiefereinrichtung aus einem Material, insbesondere Kunststoff, aufgebaut, wobei vorzugsweise die Führung in die Oberkiefereinrichtung oder die Unterkiefereinrichtung einpolymerisiert ist. Hierdurch ergibt sich eine hohe Stabilität der Vorrichtung bei geringem Herstellungsaufwand.

Gemäß einer weiteren vorteilhaften Ausgestaltung ist die Einstelleinrichtung eine Stellschraube. Dabei verläuft die Längsachse der Stellschraube zumindest im Wesentlichen parallel zu der mesialen Richtung bzw. distalen Richtung. In einer bevorzugten Variante ist die mesiale Richtung bzw. die distale Richtung bezogen auf einen Zahn, welcher in der Nähe des Stützbereichs liegt, der das mit der Stellschraube verstellbare Magnetelement aufweist. Auf diese Weise lässt sich mit der Ausrichtung der Stellschraube erreichen, dass das verstellbare Magnetelement über einen im Vergleich zu anderen Ausrichtungen der Stellschraube großen Bereich bewegt werden kann, ohne dabei in bukkaler Richtung oder entgegen der bukkalen Richtung über die Oberkiefereinrichtung oder Unterkiefereinrichtung herauszuragen. Insbesondere folgt die Bewegung beim Einstellen im Verlauf der Oberkiefereinrichtung oder der Unterkiefereinrichtung. Schließlich lässt sich auf diese Weise auch ein Herausragenden der Stellschraube selbst vermeiden. Dies erhöht insbesondere den Tragekomfort und die Sicherheit. Vorzugsweise ist die Stellschraube mit dem zugehörigen Magnetelement mittels eines Gegengewindes, insbesondere mittels einer am zugehörigen Magnetelement angebrachten Schraubenmutter, verbunden und um ihre Längsachse frei drehbar gelagert.

Gemäß einer weiteren vorteilhaften Ausgestaltung der Vorrichtung ist die Einstelleinrichtung eine Stellschraube. Zudem zeigt der Kopf der Stellschraube zumindest im Wesentlichen in mesiale Richtung. Insbesondere zeigt der Kopf der Stellschraube zumindest im Wesentlichen in einen Bereich, welcher wenigstens im Wesentlichen einem Abschnitt bei oder zwischen den Schneidezähnen des Oberkiefers und des Unterkiefers entspricht. Durch die Ausrichtung der Schraube mit in mesialer Richtung zeigenden Kopf ist es insbesondere möglich und vorteilhaft, die Stellschraube auch dann zu betätigen, wenn diese vorteilhafte Ausgestaltung an den Kiefer gekoppelt ist. Insbesondere lässt sich so die Position des verstellbaren Magneten einfach und wiederholt anpassen, bis eine gewünschte, d.h. insbesondere angenehme und/oder für die Behandlung erforderliche Position, erreicht ist, ohne dafür die Vorrichtung entnehmen zu müssen.

Gemäß einer weiteren vorteilhaften Ausgestaltung ist die Einstelleinrichtung eine Stellschraube. Zudem ist die Stellschraube mit dem zugehörigen Magnetelement mittels eines Gegengewindes verbunden und um ihre Längsachse frei drehbar gelagert. In einer bevorzugten Variante ist das Gegengewinde als eine am zu-gehörigen Magnetelement angebrachte Schraubenmutter ausgebildet. Diese vorteilhafte Ausgestaltung ermöglicht insbesondere eine einfache Herstellung.

Gemäß einer weiteren vorteilhaften Ausgestaltung ist wenigstens ein Magnetelement bzw. wenigstens ein Permanentmagnet mit einem Kunststoff beschichtet. In einer bevorzugten Variante besteht die Beschichtung aus einem Epoxidharz. In einer weiteren bevorzugten Variante die Beschichtung in der Weise ausgebildet, um das Magnetelement bzw. den Permanentmagnet vollständig einzukapseln. In einer weiteren bevorzugten Variante ist die Beschichtung dünner als 10 µm, bevorzugt dünner als 5 µm und insbesondere etwa 1 µm dick. Ein Vorteil der Beschichtung liegt insbesondere in dem Schutz des Magnetelements bzw. das Permanentmagneten. Auch lässt sich so ein Abgeben unerwünschter Stoffe verhindern. Schließlich lässt sich ein derartig beschichtetes Magnetelement bzw. ein derartig beschichteter Permanentmagnet besonders einfach verarbeiten. So können insbesondere weitere Teile, etwa eine Schraubenmutter, haltbar und stabil angeklebt werden.

Gemäß einer ersten vorteilhaften Ausgestaltung der Vorrichtung sind die Oberkiefereinrichtung und Unterkiefereinrichtung im Wesentlichen U-förmig ausgebildet sind und die jeweiligen Stützbereiche der Oberkiefereinrichtung (und/oder der Unterkiefereinrichtung über den Bereich, welcher etwa der Mitte des Kiefers entspricht, miteinander verbunden. Insbesondere sind die Oberkiefereinrichtung und/oder der Unterkiefereinrichtung gaumenfrei ausgebildet, d.h. diese weisen keine Elemente, insbesondere keine Verbindung der Stützbereiche auf, welche quer durch den Gaumen verlaufen. Dies ist für einen Träger bzw. Nutzer der Vorrichtung besonders angenehm, da keine die Zunge störenden Teile im Gaumen vorhanden sind.

Gemäß einer weiteren vorteilhaften Ausgestaltung ist die definierte Stellung des Kiefers eine Protrusion des Kiefers. Insbesondere ist die Protrusion so weit ausgeführt, dass eine Kopfbiss-Stellung des Kiefers vorliegt. Zur Behandlung von Schnarchen ist die Protrusion besonders vorteilhaft. Auf diese Weise lässt sich die Zunge in protrusiver Richtung ziehen und im Rachen bzw. bei dem Atemweg im Rachen ein größerer Freiraum schaffen und somit das Risiko und/oder die Stärke des Schnarchens senken. Schließlich ist insbesondere die Kopfbiss-Stellung zur Behandlung von Schnarchen vorteilhaft, da der Unterkiefer weit nach vorne, d.h. in protrusiver Richtung, eingestellt und so ein Freiraum für die Zunge geschaffen wird. Zusätzlich erlaubt die Kopfbiss-Stellung zusammen mit der Lagerung und dem Abstützen im Bereich des sinistralen und dextralen Hauptkauzentrums, insbesondere die Ruhe-Schwebe des Unterkiefers und damit, eine Entspannung der Kiefermuskulatur und eine Entlastung des Kiefergelenks. Abschließend wird damit insbesondere ein verbesserter Tragekomfort und ein verstärkter Behandlungseffekt erzielt.

Gemäß einer weiteren vorteilhaften Ausgestaltung ist die Vorrichtung dazu ausgebildet, von einem Menschen getragen zu werden. Bevorzugt besteht diese Ausgestaltung nur aus biokompatiblen, insbesondere medizinisch zugelassenen, Materialien und/oder kapselt andere Materialien, etwa mittels einer Beschichtung, sicher ein.

Gemäß einer weiteren vorteilhaften Ausgestaltung ist die Vorrichtung dazu aus-gebildet, von einem Säugetier, etwa einem Hund oder einem Pferd, getragen zu werden. Da eine erfindungsgemäße Vorrichtung keine Zwangsstellung bedingt, ist diese besonders zur Behandlung von Säugetieren geeignet. Insbesondere wird das Vermeiden einer Zwangsstellung die Akzeptanz durch einen Träger erhöht. Dies kann in einem gesteigerten Behandlungseffekt resultieren.

Weitere Vorteile, Merkmale und Anwendungsmöglichkeiten der vorliegenden Erfindung ergeben sich aus der nachfolgenden detaillierten Beschreibung im Zusammenhang mit den Figuren.

Dabei zeigt
- **Fig. 1**: einen Kiefer mit Oberkiefer und Unterkiefer sowie Zähnen als Darstellung von der dextralen Seite und von der sinistralen Seite zusammen mit Richtungsangaben;
- **Fig. 2**: eine wenigstens teilweise schematische Seitenansicht einer bevorzugten Ausführungsform der erfindungsgemäßen Vorrichtung, welche an die Zähne des Kiefers gekoppelt ist und eine definierte Stellung des Kiefers einstellt; und
- **Fig. 3**: eine wenigstens teilweise schematische Draufsicht einer Oberkiefereinrichtung und eine Unterkiefereinrichtung einer bevorzugten Ausführungsform der erfindungsgemäßen Vorrichtung.

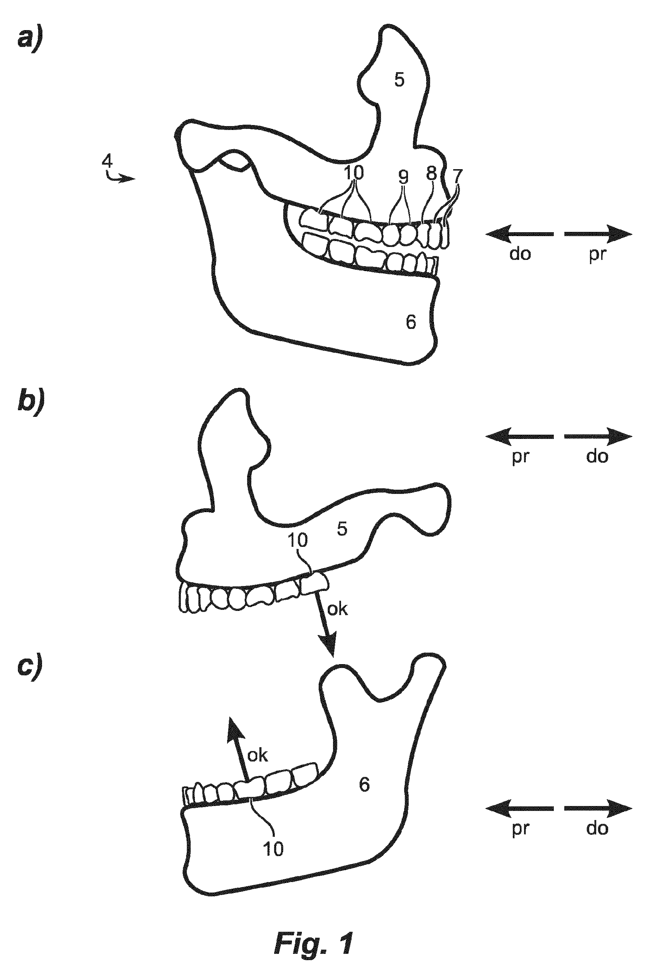

Zunächst wird auf **Fig. 1** Bezug genommen, in der ein Kiefer 4 mit Oberkiefer 5 und Unterkiefer 6 sowie Zähnen 7, 8, 9, 10 skizziert ist. In Fig. 1 a ist der Kiefer 4 von der dextralen Seite dargestellt, in Fig. 1 b der Oberkiefer 5 von der sinistralen Seite und in Fig. 1 c der Unterkiefer 6 von der sinistralen Seite.

Im Folgenden werden die Nummern für einzelne Zähne 7, 8, 9, 10 gemäß dem FDI-Zahnschema mit den jeweiligen Bezugszeichen in Verbindung gesetzt. Auf der dextralen Seite des Oberkiefers 5 haben die Schneidezähne 7 die Nummern 11 und 12, der Eckzahn 8 die Nummer 13, die Prämolaren 9 die Nummern 14 und 15 und die großen Backenzähne 10 bzw. Molaren 10 die Nummern 16, 17 und 18. Auf der dextralen Seite des Unterkiefers 6 haben die Schneidezähne 7 die Nummern 41 und 42, der Eckzahn 8 die Nummer 43, die Prämolaren 9 die Nummern 44 und 45 und die großen Backenzähne 10 bzw. Molaren 10 die Nummern 46, 47 und 48. Auf der sinistralen Seite des Oberkiefers 5 haben die Schneidezähne 7 die Nummern 21 und 22, der Eckzahn 8 die Nummer 23, die Prämolaren 9 die Nummern 24 und 25 und die großen Backenzähne 10 bzw. Molaren 10 die Nummern 26, 27 und 28. Auf der sinistralen Seite des Unterkiefers 6 haben die Schneidezähne 7 die Nummern 31 und 32, der Eckzahn 8 die Nummer 33, die Prämolaren 9 die Nummern 34 und 35 und die großen Backenzähne 10 bzw. Molaren 10 die Nummern 36, 37 und 38.

In **Fig. 1a** sind die protrusive Richtung pr sowie die dorsale Richtung do, insbesondere für die Bewegung des Unterkiefers 6, mit Pfeilen dargestellt. In **Fig. 1b** sind wieder die protrusiver Richtung pr sowie die dorsale Richtung do dargestellt, wobei die Pfeile in der Zeichnung aufgrund der Darstellung als sinistrale Seitenansicht in die entgegengesetzte Richtung zeigen. Zudem ist die okklusale Richtung ok für den Oberkiefer 5, insbesondere bezogen auf den Molaren 10 mit der Nummer 28 gemäß FDI-Zahnschema, mit einem Pfeil dargestellt. Schließlich ist in Fig. 1 c die protrusive Richtung pr und die dorsale Richtung do sowie die okklusale Richtung ok für den Unterkiefer 6, insbesondere bezogen auf den Molaren 10 mit Nummer 36 gemäß FDI-Zahnschema, mit je einem Pfeil dargestellt. Hierbei ist zu erkennen, dass die okklusale Richtung ok für den Oberkiefer 5 und den Unterkiefer 6 zumindest im Wesentlichen entgegengesetzt sind und zudem auch einen von 180° abweichenden Winkel einschließen können, insbesondere wenn diese auf Zähne 7, 8, 9, 10 bezogen sind, welche nicht gleich bzw. nicht genau entgegengesetzt ausgerichtet sind.

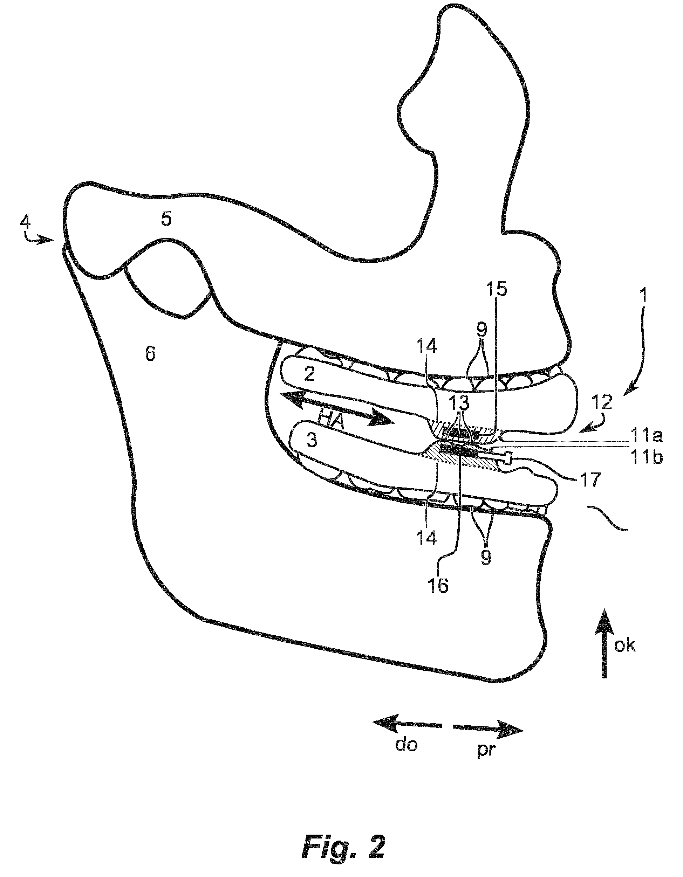

**Fig. 2** zeigt schematisch eine dextrale Seitenansicht einer bevorzugten Ausführungsform der erfindungsgemäßen Vorrichtung 1, welche eine definierte Stellung des Kiefers 4 einstellt. Dabei ist die Oberkiefereinrichtung 2 an die Zähne 7, 8, 9, 10 des Oberkiefers 5 gekoppelt und die Unterkiefereinrichtung 3 an die Zähne 7, 8, 9, 10 des Unterkiefers 6. Zudem ist die hier dargestellte bevorzugte Ausführungsform insbesondere für eine Protrusion des Unterkiefers 6 ausgelegt und die definierte Stellung des Kiefers ist eine Protrusion, insbesondere eine Kopfbiss-Stellung. Die hier dargestellte bevorzugte Ausführungsform ist vorzugsweise mit einer Tiefziehfolie gefertigt. Diese lässt sich mittels eines Positivmodells jeweils der Zähne des Oberkiefers und des Unterkiefers formen und ermöglicht so eine formschlüssige und/oder kraftschlüssige Kopplung an die Zähne des Kiefers. Speziell wird hierfür eine Tiefziehfolie mit einer Materialstärke zwischen 1 mm und 3 mm, insbesondere 2 mm, eingesetzt. Für einen erhöhten Tragekomfort kann dabei insbesondere die erfindungsgemäße Vorrichtung 1 in der Weise geformt sein, um möglichst wenig Material um die Zähne herum aufzutragen. Insbesondere kann so ein mögliches Fremdkörpergefühl verringert, der Zunge ein größtmöglicher Freiraum gegeben sowie ein Druck auf die Backen und/oder Lippen vermieden werden.

Als Material für die Tiefziehfolie kommen insbesondere Thermoplasten oder andere Kunststoffarten in Frage, welche im erhärteten Zustand halb-hart sind und vorzugsweise eine Resilienz aufweisen, um über den Äquator der Zähne geschoben und auch wieder entfernt werden können. Weiter vorzugsweise verbindet sich das Material gut mit insbesondere transparentem Prothesenkunststoff. Insbesondere kommt das Material Erkodur® der Fa. Erkodent als Material in Frage. Der Durchmesser des Materialstücks beträgt vorzugsweise 120mm, die Materialdicke weiter vorzugsweise 2mm.

Zumindest in der definierten Stellung des Kiefers 4 grenzen die sich paarweise gegenüberliegenden Stützbereiche 11a, 11 b mit Stützflächen 13 aneinander an. Diese Stützflächen 13 sind auf Stützeinrichtungen 14 angeordnet, welche in Fig. 2 als seitlicher Querschnitt illustriert sind. Insbesondere weist die Oberkiefereinrichtung 2 eine Stützeinrichtung 14 mit einer Stützfläche 13 auf und die Unterkiefereinrichtung 3 eine Stützeinrichtung 14 mit zwei Stützflächen 13. Bevorzugt erstreckt sich die jeweilige Stützfläche 13 bei aneinander angrenzenden Stützbereichen 11, 11a, 11 b, insbesondere bei der definierten Stellung des Kiefers 4, räumlich in jeder Dimension um höchstens 3 cm, bevorzugt um höchstens 2 cm, weiter bevorzugt um höchstens 1 cm und weist insbesondere eine Fläche im Bereich von 0,5 cm² bis 3 cm² auf. Dies ist insbesondere wichtig um eine Lagerung des Unterkiefers 6 im Hauptkauzentrum zu ermöglichen. Bei mehreren Stützflächen 13 eines Stützbereichs 11, die zeitgleich auftreten, soll bevorzugt das Vorangegangene auch für die Einhüllende der mehreren Stützflächen 13 gelten. In Fig. 2 ist zu erkennen, dass die Stützeinrichtungen 14 jeweils etwa im Bereich der Prämolaren 9 angeordnet sind. Speziell die Stützeinrichtung 14, welche die Unterkiefereinrichtung 3 aufweist, ist im Vergleich zur gegenüberliegenden Stützeinrichtung 14 des Oberkiefers 5 in dorsaler Richtung do bzw. in Richtung der Molaren 10 bzw. Backenzähne 10 verschoben. Hierdurch lässt sich eine weiter verbesserte Lagerung des Unterkiefers 6 im Hauptkauzentrum erzielen. Überdies ist hier gezeigte die Anordnung der Stützeinrichtungen 14 insbesondere vorteilhaft, um eine Protrusion des Unterkiefers 6, speziell bis zur Kopfbiss-Stellung, zu erreichen.

Außerdem sind die in Fig. 2 dargestellten Stützbereiche 11 so ausgebildet, dass auch bei aneinander angrenzenden Stützbereichen 11 die Oberkiefereinrichtung 2 und die Unterkiefereinrichtung 3 relativ zueinander zumindest im Wesentlichen entlang der Hauptachse HA verschiebbar sind. Dabei können je nach relativer Verschiebung verschiedene Stützflächen 13 der Stützeinrichtungen 11 aneinander angrenzen. Insbesondere wird durch das Aneinanderangrenzen ein weiteres Schließen des Kiefers 4, also speziell ein Bewegen des Unterkiefers 6 in okklusaler Richtung ok, verhindert und die Unterkiefereinrichtung 3 gegen die Oberkiefereinrichtung 2 abgestützt.

Die Oberkiefereinrichtung 2 und die Unterkiefereinrichtung 3 der in Fig. 2 dargestellten, bevorzugten Ausführungsform der erfindungsgemäßen Vorrichtung 1 sind so ausgeformt, dass bei aneinander angrenzenden Stützbereichen 11 eine Öffnung für Atemluft 12 im Bereich zwischen den Schneidezähnen 7 des Oberkiefers 5 und des Unterkiefers 6 ausgebildet ist. Bevorzugt sind dabei die Oberkiefereinrichtung 2 und die Unterkiefereinrichtung 3 so ausgeformt, dass die Öffnung für Atemluft 12 möglichst groß ist. Dazu werden diese insbesondere im Bereich der Schneidezähne 7 in möglichst geringen Materialstärken und/oder mit Aussparungen gefertigt. Speziell bei der bevorzugten Ausführungsform, welche mit Tiefziehfolie gefertigt ist, kann dort eine Materialstärke von weniger als 3 mm, bevorzugt weniger als 2 mm verwendet werden und/oder die Tiefziehfolie so geformt sein, dass diese an den Zähnen anliegt und den Raum zwischen den Schneidezähnen 7 des Oberkiefers 5 und des Unterkiefers 6 nur etwa um die eigene Materialstärke verkleinert.

Die in Fig. 2 als seitlicher Querschnitt illustrierte Stützeinrichtung 14 der Oberkiefereinrichtung 2 weist ein Magnetelement 15 auf und die seitlicher Querschnitt illustrierte Stützeinrichtung 14 der Unterkiefereinrichtung 3 weist ein Magnetelement 16 auf. Bevorzugt sind in der hier dargestellten bevorzugten Ausführungsform die Magnetelemente 15, 16 als Permanentmagneten ausgebildet. Diese können etwa quaderförmig sein sowie eine räumliche Ausdehnung von 10 mm in protrusiver Richtung pr, 1 mm in okklusaler Richtung ok und 5 mm in bukkaler Richtung bu aufweisen. Bevorzugt sind diese in okklusaler Richtung ok polarisiert, wobei die Polarität in der Weise ausgebildet ist, um sich bei aneinander angrenzenden Stützbereichen, insbesondere bei der definierten Stellung des Kiefers, magnetisch anzuziehen. Zudem bewirken die Magnetelemente 15, 16 insbesondere eine Kraft in Richtung der definierten Stellung des Kiefers 4. Sofern die Unterkiefereinrichtung 3 relativ zur Oberkiefereinrichtung 2 aus der definierten Stellung des Kiefers 4 heraus, etwa entlang der Hauptachse HA, verschoben worden ist, kann die Kraft eine Rückführung in die definierte Stellung des Kiefers 4 bewirken. Umgekehrt ist die Magnetkraft der Magnetelemente 15, 16 in der Weise dimensioniert, um ein Verschieben aus der definierten Stellung des Kiefers 4 heraus und/oder ein Öffnen des Kiefers 4, d.h. ein Bewegen des Unterkiefers 6 entgegen der okklusalen Richtung ok, durch Muskelkraft, insbesondere der Kiefermuskulatur, zu ermöglichen. Dazu weisen die Magnetelemente, insbesondere die Permanentmagneten 15, 16, eine Magnetisierung auf, die jeweils in einer Abzugskraft von etwa 600 Gramm bzw. etwa 6 N resultiert.

Schließlich weist die in Fig. 2 dargestellte Stützeinrichtung 14 der Unterkiefereinrichtung 3 eine Stellschraube 17 auf mit der das Magnetelement dieser Stützeinrichtung 14 entlang der Hauptachse HA verschoben werden kann. Auch bevorzugt kann die Stellschraube 17 so angeordnet und ausgebildet sein, dass das Magnetelement 16 in mesialer und/oder distaler Richtung verschiebbar ist. Bevorzugt ist die Stellschraube in der gezeigten Ausführungsform aus Titan gefertigt und weist ebenfalls bevorzugt einen Durchmesser von etwa 1 mm auf. In der hier gezeigten bevorzugten Ausführungsform weißt der Schraubenkopf 19 zudem in mesiale Richtung me oder in Richtung eines Bereichs, welcher wenigstens im Wesentlichen einem Abschnitt zwischen den Schneidezähnen 7 des Oberkiefers 5 und des Unterkiefers 6 entspricht. Insbesondere lässt sich so das Magnetelement 16 über die Stellschraube 17 verschieben, während die Vorrichtung 1 getragen wird. Wie oben bereits beschrieben sind die Magnetelemente 15, 16 bevorzugt in der Weise ausgebildet, um eine Kraft in Richtung der definierten Stellung des Kiefers 4 zu bewirken. Die Magnetelemente 15, 16 ziehen sich insbesondere so an, dass diese in Deckung miteinander kommen. Ein Verschieben des Magnetelements 16 der Unterkiefereinrichtung 3 bewirkt insbesondere eine Kraft, welche der Verschiebung entgegen wirkt und bei Bewegung in Richtung der Kraft die Magnetelemente wieder in Deckung bringt. Folglich lässt sich die definierte Stellung des Kiefers 4 über die Stellschraube 17 anpassen. Bevorzugt kann dabei der maximal mögliche Bereich aufgrund physiologischer, insbesondere kiefergelenksbezogener, Überlegungen durch einen Fachmann bereits über die Ausbildung der Ausführungsform vorgegeben sein. Weiterhin bevorzugt kann die erfindungsgemäße Vorrichtung 1 bzw. die durch diese eingestellte definierte Stellung des Kiefers 4 dann im Gebrauch angepasst werden. Insbesondere lässt sich auf diese Weise der Grad der Protrusion des Unterkiefers 6 einstellen. So kann ein Mensch, der die hier dargestellte bevorzugte Ausführungsform zur Behandlung von Schnarchen trägt den Grad der Protrusion in der Weise variieren, dass einerseits das Schnarchen verhindert oder zumindest verringert wird und andererseits ein angenehmes Tragen, insbesondere durch Abstützen und Lagern des Unterkiefers 6 im Bereich des Hauptkauzentrums, erreicht wird

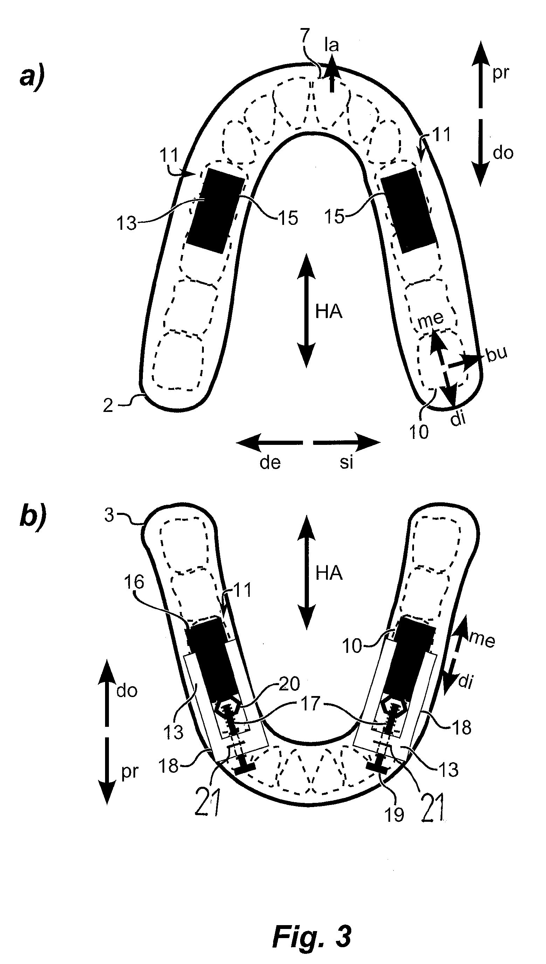

**Fig. 3a** skizziert eine Draufsicht einer Oberkiefereinrichtung 2 einer bevorzugten Ausführungsform der erfindungsgemäßen Vorrichtung 1. Dabei sind die Bereiche, welche zur Kopplung mit den Zähnen 7, 8, 9, 10 des Oberkiefers 5 ausgeformt sind, vom Betrachter abgewandt und die Stützbereiche 11 dem Betrachter zugewandt. Außerdem sind als Strichzeichnungen die korrespondierenden Positionen der Zähne 7, 8, 9, 10 des Oberkiefers 5 angedeutet.

Durch Pfeile sind die protrusiver Richtung pr, die dorsale Richtung do, die dextrale Richtung de und die sinistrale Richtung si, welche zumindest im Wesentlichen in der Zeichenebene liegen, aufgezeigt. Auch die Hauptachse HA liegt zumindest im Wesentlichen in der Zeichenebene und verläuft insbesondere entlang der protrusiven Richtung pr sowie der dorsalen Richtung do. Weiterhin sind die mesiale Richtung me, die distale Richtung di und die bukkale Richtung bu bezüglich des Molaren 10 mit der Nummer 28 gemäß FDI-Zahnschema illustriert. Die okklusale Richtung ok bezüglich dieses Molaren 10 zeigt im Wesentlichen in Richtung des Betrachters und ist somit hier nicht illustriert. Schließlich ist die labiale Richtung la bezüglich des Schneidezahnes 7 mit der Nummer 21 gemäß FDI-Zahnschema illustriert.

Die Stützflächen 13 sind in der hier dargestellten bevorzugten Ausführungsform auf den Magnetelementen 15 angeordnet. Die Magnetelemente 15 weisen bevorzugt eine Beschichtung mit einem Kunststoff, insbesondere mit einem Epoxidharz, auf. Diese Beschichtung ist vorzugsweise so ausgebildet, dass das Magnetelement vollständig eingekapselt ist. Zudem ist die Beschichtung vorzugsweise dünner als 10 µm, bevorzugt dünner als 5 µm und insbesondere etwa 1 µm dick. Die Magnetelemente 15 sind mit der Oberkiefereinrichtung 2, vorzugsweise stoffschlüssig und im Wesentlichen unbeweglich, verbunden. Insbesondere lässt sich durch die Epoxidbeschichtung eine verbesserte stoffschlüssige Verbindung, insbesondere ein haltbares Verkleben, erreichen. Auch sind die Magnetelemente 15 insbesondere im Bereich der Prämolaren 9 angeordnet und in mesialer me bzw. distaler Richtung di ausgerichtet. Schließlich sind die Magnetelemente 15 vorzugsweise als quaderförmige Permanentmagneten 15 ausgebildet und weisen jeweils eine räumliche Ausdehnung von 10 mm mesialer/distaler Richtung me/di, 5 mm bukkaler Richtung bu und 1 mm in okklusaler Richtung ok auf.

**Fig. 3b** skizziert eine Draufsicht einer Unterkiefereinrichtung 3 einer bevorzugten Ausführungsform der erfindungsgemäßen Vorrichtung 1. Dabei sind die Bereiche, welche zur Kopplung mit den Zähnen 7, 8, 9, 10 des Unterkiefers 6 ausgeformt sind, vom Betrachter abgewandt und die Stützbereiche 11 dem Betrachter zugewandt. Außerdem sind als Strichzeichnungen die korrespondierenden Positionen der Zähne 7, 8, 9, 10 des Unterkiefers 6 angedeutet.

Durch Pfeile sind die protrusiver Richtung pr und die dorsale Richtung, welche zumindest im Wesentlichen in der Zeichenebene liegen, aufgezeigt. Weiterhin sind die mesiale Richtung me und die distale Richtung di bezüglich des Molaren 10 mit der Nummer 36 gemäß FDI-Zahnschema illustriert. Die okklusale Richtung ok bezüglich dieses Molaren zeigt im Wesentlichen in Richtung des Betrachters und ist somit hier nicht illustriert.

Die Magnetelemente 16 entsprechen im Wesentlichen denen aus der vorangegangenen Fig. 3a und sind vorzugsweise beschichtet sowie in mesialer me bzw. distaler Richtung di ausgerichtet. Jedoch sind die Magnetelemente 16 nicht unbeweglich mit der Unterkiefereinrichtung 3 verbunden. Die Unterkiefereinrichtung 3 weist zwei symmetrisch angeordnete Stützeinrichtungen 14 mit formschlüssigen Führungen 18 auf, die vorzugsweise als Schienen ausgeführt sein. Die Magnetelemente 16 werden in den formschlüssigen Führungen bzw. Schienen 18 geführt und sind in mesialer me und distaler Richtung di beweglich. Dabei wird insbesondere eine Bewegung der Magnetelemente 16 in und entgegen der okklusalen Richtung ok verhindert. Mit den Magnetelementen 16 ist jeweils eine Schraubenmutter 20 stoffschlüssig verbunden. Insbesondere kann diese an die Magnetelemente 15, 16, insbesondere bei epoxidbeschichteten Magnetelementen 15,16 angeklebt sein. In die Schraubenmutter 20, welche ebenfalls aus Kunststoff, insbesondere Epoxid bestehen kann, greift jeweils eine Stellschraube 17 ein, die um ihre Längsachse frei drehbar in der jeweiligen Stützeinrichtung 14 gelagert ist und vorzugweise aus Titan besteht.

Durch ein Drehen der Stellschraube 17 lässt sich das jeweilige Magnetelement 16 in mesialer Richtung me und in distaler Richtung di verschieben, wobei die Stellschraube 17 gegenüber den Führungen 18, vorzugsweise durch einen Hals 21, welcher in den Führungen gelagert ist, abgestützt wird. Der maximal mögliche Verschiebebereich kann dabei bevorzugt über einen Anschlag an der Stützeinrichtung 14 und/oder das Gewinde der Stellschraube 17 vorgegeben werden. Wie hier gezeigt weißt der Kopf 19 bevorzugt in mesiale Richtung me. Auf diese Weise lässt sich das Magnetelement 16 verschieben während die Unterkiefereinrichtung 3 getragen wird bzw. an die Zähne 7, 8, 9, 10 des Unterkiefers 6 gekoppelt ist. Insbesondere treffen die dazu bei Fig. 2 genannten Vorteile auch hier zu. Insbesondere kann die Führung 18 als Kasten bzw. Gehäuse, insbesondere Kunststoffkasten ausgebildet sein, welcher die Magnetelemente 15, 16 umgibt. Der Kasten ist dabei in Verschieberichtung des Magnetelements 15 länger als das Magnetelement 15,16 bzw. das Magnetelement samt Schraubenmutter 20, vorzugsweise etwa 5 mm länger, weiter vorzugsweise etwa 8 bis 10 mm länger, und weist vorzugsweise allenfalls eine Öffnung auf, durch welche die Stellschraube 17 geführt wird. Die Führung 18 ist vorzugsweise aus Kunststoff und insbesondere aus demselben Material wie die Unterkiefereinrichtung 3 und/oder die Oberkiefereinrichtung 2. Insbesondere ist die Führung 18 mit der Unterkiefereinrichtung 3 und/oder die Oberkiefereinrichtung 2 verklebt, vorzugsweise in diese einpolymerisiert.

Schließlich sind die Stützbereiche 11 bei Fig. 3b über die Stützeinrichtungen 14 definiert und die Stützflächen 13 sind auf den formschlüssigen Führungen 18 angeordnet. Bei getragener Oberkiefereinrichtung 2 gemäß Fig. 3a und getragener Unterkiefereinrichtung 3 gemäß Fig. 3b liegen sich die Magnetelemente 15 der Oberkiefereinrichtung 2 und die Stützeinrichtungen 14 der Unterkiefereinrichtung 3 jeweils paarweise gegenüber. Insbesondere grenzen zumindest in der definierten Stellung des Kiefers 4 die auf den Magnetelementen 15 der Oberkiefereinrichtung 2 angeordneten Stützflächen 13 an die auf den formschlüssigen Führungen 18 angeordneten Stützflächen 13 aneinander an. Bevorzugt sind die gegenüberliegenden Oberflächen der Magnetelemente 15 und der formschlüssigen Führungen 18 im Wesentlichen glatt ausgeführt, um die Verschiebbarkeit in protrusiver Richtung pr und in dorsaler Richtung do zu verbessern.

Daneben ist es insbesondere auch möglich und ebenfalls bevorzugt die Magnetelemente 15 der Oberkiefereinrichtung 2 beweglich an der Oberkiefereinrichtung 2 anzubringen und/oder die Magnetelemente 16 der Unterkiefereinrichtung 3 unbeweglich an der Unterkiefereinrichtung 3 anzubringen. Auch ist es bevorzugt nur ein Paar der Magnetelemente 15, 16, insbesondere nur ein Magnetelement, beweglich anzubringen. Auf diese Weise lässt sich die definierte Stellung des Kiefers 4 bereits mit nur einer Stellschraube 17 vollständig anpassen.

Während vorausgehend wenigstens eine beispielhafte Ausführungsform beschrieben wurde, ist zu bemerken, dass eine große Anzahl von Variationen dazu existiert. Es ist dabei auch zu beachten, dass die beschriebenen beispielhaften Ausführungsformen nur nichtlimitierende Beispiele darstellen, und es nicht beabsichtigt ist, dadurch den Umfang, die Anwendbarkeit oder die Konfiguration der hier beschriebenen Vorrichtungen und Verfahren zu beschränken. Vielmehr wird die vorausgehende Beschreibung dem Fachmann eine Anleitung zur Implementierung mindestens einer beispielhaften Ausführungsform liefern, wobei es sich versteht, dass verschiedene Änderungen in der Funktionsweise und der Anordnung der in einer beispielhaften Ausführungsform beschriebenen Elemente vorgenommen werden können, ohne dass dabei von dem in den angehängten Ansprüchen jeweils festgelegten Gegenstand sowie seinen rechtlichen Äquivalenten abgewichen wird.

### Bezugszeichenliste

- 1: Erfindungsgemäße Vorrichtung
- 2: Oberkiefereinrichtung
- 3: Unterkiefereinrichtung
- 4: Kiefer
- 5: Oberkiefer
- 6: Unterkiefer
- 7: Schneidezahn
- 8: Eckzahn
- 9: Backenzahn, insbesondere Prämolar
- 10: Backenzahn, insbesondere Molar
- 11: Stützbereich
- 11a, 11b: Paar sich anziehender bzw. gegenüberliegender Stützbereiche mit je einem bei der Oberkiefereinrichtung und der Unterkiefereinrichtung angeordneten Stützbereich
- 12: Öffnung für Atemluft
- 13: Stützfläche
- 14: Stützeinrichtung
- 15: Magnetelement, insbesondere von der Oberkiefereinrichtung, insbesondere aufweisend einen Permanentmagnet
- 16: Magnetelement, insbesondere von der Unterkiefereinrichtung
- 17: Einstelleinrichtung, insbesondere ausgebildet als Stellschraube
- 18: Formschlüssige Führung, insbesondere ausgebildet als Schiene
- 19: Kopf einer Stellschraube
- 20: Gegengewinde, insbesondere ausgebildet als Schraubenmutter
- 21: Hals

- HA: Hauptachse
- bu: Bukkale Richtung
- de: Dextrale Richtung
- di: Distale Richtung
- do: Dorsale Richtung
- la: Labiale Richtung
- me: Mesiale Richtung
- ok: Okklusale Richtung
- pr: Protrusive Richtung
- si: Sinistrale Richtung

## Patentansprüche

1. Vorrichtung (1) zum Einstellen einer definierten Stellung eines Kiefers (4), insbesondere zur Behandlung von Schnarchen, aufweisend
eine Oberkiefereinrichtung (2), welche in der Weise ausgebildet ist, um wenigstens teilweise an Zähne (7, 8, 9, 10) eines Oberkiefers (5), insbesondere formschlüssig und/oder kraftschlüssig, gekoppelt zu werden und
eine Unterkiefereinrichtung (3), welche in der Weise ausgebildet ist, um wenigstens teilweise mit Zähnen (7, 8, 9, 10) eines Unterkiefers (6), insbesondere formschlüssig und/oder kraftschlüssig, gekoppelt zu werden,
wobei die Oberkiefereinrichtung (2) und die Unterkiefereinrichtung (3) jeweils, insbesondere genau, zwei Stützbereiche (11) aufweisen, welche, insbesondere im Wesentlichen symmetrisch, auf beiden Seiten eines Bereichs, welcher etwa der Mitte des Kiefers (4) entspricht, insbesondere im Abschnitt der Prämolaren (9) des Kiefers (4), angeordnet sind,
wobei jeweils ein Paar gegenüberliegender Stützbereiche (11 a, 11 b) der Oberkiefereinrichtung und (2) der Unterkiefereinrichtung (3) in der Weise ausgebildet sind, um wenigstens in der definierten Stellung des Kiefers (4) aneinander anzugrenzen, und sich wenigstens eines der Paare (11a, 11b), vorzugsweise beide Paare, magnetisch anzieht, und
wobei die Stützbereiche (11) in der Weise ausgebildet sind, dass die Oberkiefereinrichtung (2) und die Unterkiefereinrichtung (3) bei aneinander angrenzenden Stützbereichen (11, 11a, 11 b) wenigstens im Wesentlichen entlang einer Hauptachse (HA) der Vorrichtung (1) relativ zueinander verschiebbar sind.

2. Vorrichtung gemäß Anspruch 1, wobei die Stützbereiche (11) in jeweils einem Bereich angeordnet sind, welche einem Abschnitt der Prämolaren des Kiefers (4) entsprechen.

3. Vorrichtung (1) gemäß Anspruch 1 oder 2, wobei eine magnetische Anziehungskraft des wenigstens einen Paars gegenüberliegender Stützbereiche (11a, 11b) so eingerichtet ist, um eine Verschiebung und/oder eine Trennung der Oberkiefereinrichtung (2) und der Unterkiefereinrichtung (3) durch Muskelkraft, insbesondere der Kiefermuskulatur, zu ermöglichen.

4. Vorrichtung (1) gemäß einem der vorausgehenden Ansprüche, wobei beide Paare aneinander angrenzender Stützbereiche (11, 11a, 11 b) in der Weise ausgebildet sind, um die Oberkiefereinrichtung (2) und die Unterkiefereinrichtung (3) in einem definierten Abstand, welcher etwa 10 mm bis 2 mm, bevorzugter 8 mm bis 4 mm, und am bevorzugtesten 6 mm beträgt, gegeneinander abzustützen.

5. Vorrichtung (1) gemäß einem der vorausgehenden Ansprüche, wobei die Vorrichtung (1) bei aneinander angrenzenden Stützbereichen (11, 11a, 11b) wenigstens eine Öffnung für Atemluft (12) zwischen den Stützbereichen (11, 11a, 11b) aufweist, insbesondere in einem Bereich, welcher wenigstens im Wesentlichen einem Abschnitt zwischen den Schneidezähnen (7) des Oberkiefers (5) und des Unterkiefers (6) entspricht, wobei die wenigstens eine Öffnung (12) vorzugsweise zwischen der Oberkiefereinrichtung (2) und der Unterkiefereinrichtung (3) ausgebildet ist.

6. Vorrichtung (1) gemäß einem der vorausgehenden Ansprüche, die Oberkiefereinrichtung (2) und die Unterkiefereinrichtung (3) wenigstens in beide Richtungen entlang der Hauptachse (HA) ausgehend von der definierten Stellung des Kiefers um etwa 1 mm, bevorzugt um wenigstens 1,5 mm, bevorzugter um wenigstens 5 mm und weiter bevorzugt um wenigstens 15 mm relativ zueinander durch Muskelkraft, insbesondere der Kiefermuskulatur, verschiebbar sind.

7. Vorrichtung (1) gemäß einem der vorausgehenden Ansprüche, wobei an dem wenigstens einen Paar sich anziehender Stützbereiche (11 a, 11 b) jeweils Magnetelemente (15, 16) in der Weise angeordnet sind, um zwischen der Oberkiefereinrichtung (2) und der Unterkiefereinrichtung (3) eine Kraft in Richtung der definierten Stellung des Kiefers (4) zu bewirken, wobei die Magnetelemente (15, 16) jeweils vollständig von der der Oberkiefereinrichtung (2) oder der Unterkiefereinrichtung (3) umschlossen werden.

8. Vorrichtung (1) gemäß einem der vorausgehenden Ansprüche,
wobei die Stützbereiche (11) jeweils wenigstens eine Stützfläche (13) aufweisen, und
wobei jedes Paar sich anziehender Stützbereiche (11a, 11b) wenigstens ein Magnetelement (15, 16) aufweist, dessen Position in einer durch die jeweilige wenigstens eine Stützfläche (13) definierten Ebene verstellbar ist, wobei eine Verstellrichtung insbesondere wenigstens eine Komponente in Richtung der Hauptachse (HA) und/oder wenigstens in Richtung senkrecht zu dieser und/oder wenigstens in mesialer Richtung (me) und/oder wenigstens in distaler Richtung (di) aufweist.

9. Vorrichtung (1) gemäß einem der vorausgehenden Ansprüche, wobei die Oberkiefereinrichtung (2) und/oder die Unterkiefereinrichtung (3) wenigstens eine Einstelleinrichtung (17), insbesondere eine Stellschraube, zum Verstellen der Position des wenigstens einen Magnetelements (15, 16) aufweist, mit welcher die definierte Stellung des Kiefers (4) eingestellt werden kann.

10. Vorrichtung (1) gemäß einem der vorausgehenden Ansprüche, wobei die Vorrichtung (1), insbesondere die Unterkiefereinrichtung (3), wenigstens eine, insbesondere formschlüssige, Führung (18), insbesondere eine Schiene und/oder einen Kasten, zur Führung des wenigstens einen Magnetelements (15, 16) aufweist.

11. Vorrichtung (1) gemäß Anspruch 10, wobei die Führung (18) das wenigstens eine Magnetelement (15, 16) bis auf eine Öffnung für die Einstelleinrichtung (17) vollständig umschließt.

12. Vorrichtung (1) gemäß Anspruch 10 oder 11, wobei die Führung (18) und die Oberkiefereinrichtung (2) und/oder die Unterkiefereinrichtung (3) aus Kunststoff aufgebaut sind, insbesondere dasselbe Material aufweisen, und wobei vorzugsweise die Führung (18) mit der Oberkiefereinrichtung (2) oder der Unterkiefereinrichtung (3) verklebt, insbesondere in diese einpolymerisiert, ist.

13. Vorrichtung (1) gemäß einem der Ansprüche 9 bis 12, wobei die Einstelleinrichtung (17) eine Stellschraube (17) ist, deren Längsachse zumindest im Wesentlichen parallel zu der mesialen Richtung (me) bzw. distalen Richtung (di) verläuft, insbesondere bezogen auf einen Zahn (7, 8, 9, 10), welcher in der Nähe des Stützbereichs (11) liegt, der das verstellbare Magnetelement (15, 16) aufweist, und wobei die Stellschraube (17) mit dem zugehörigen Magnetelement (15, 16) mittels eines Gegengewindes (20), insbesondere mittels einer am zugehörigen Magnetelement angebrachten Schraubenmutter (20), verbunden und um ihre Längsachse frei drehbar gelagert ist.

14. Vorrichtung (1) gemäß Anspruch 13, wobei die Stellschraube (17) einen Hals (21) aufweist, welcher von in der Führung (18) gelagert ist, um eine axiale Verschiebung der Stellschraube (17) wenigstens im Wesentlichen in Bezug auf die Führung (18) zu verhindern.

15. Vorrichtung (1) gemäß einem der vorhergehenden Ansprüche, wobei die der Oberkiefereinrichtung (2) und der Unterkiefereinrichtung (3) im Wesentlichen U-förmig ausgebildet sind und die jeweiligen Stützbereiche der Oberkiefereinrichtung (2) und/oder der Unterkiefereinrichtung (3) über den Bereich, welcher etwa der Mitte des Kiefers (4) entspricht, miteinander verbunden sind.
